Europäisches Patentamt
European Patent Office
Office européen des brevets

(19)

(11) Veröffentlichungsnummer: **0 192 031**
**A1**

(12)

# EUROPÄISCHE PATENTANMELDUNG

(21) Anmeldenummer: 86100263.2

(22) Anmeldetag: 10.01.86

(51) Int. Cl.⁴: C 07 D 263/58
C 07 D 277/68, A 01 N 43/76
A 01 N 43/78
//C07C143/68

(30) Priorität: 24.01.85 DE 3502266

(43) Veröffentlichungstag der Anmeldung: 27.08.86 Patentblatt 86/35

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Förster, Heinz, Dr.
Am Eckbusch 47
D-5600 Wuppertal 1(DE)

(72) Erfinder: Yanagi, Akihiko
39-15, Namiki-cho
Hachioji-shi Tokyo(JP)

(72) Erfinder: Santel, Hans-Joachim, Dr.
Gruenstrasse 9a
D-5090 Leverkusen(DE)

(72) Erfinder: Schmidt, Robert R. Dr.
Im Waldwinkel 110
D-5060 Bergisch-Gladbach 2(DE)

(54) **Optisch aktive Phenoxypropionsäure-Derivate.**

(57) R-Enantiomere von Phenoxypropionsäure-Derivaten der Formel (I)

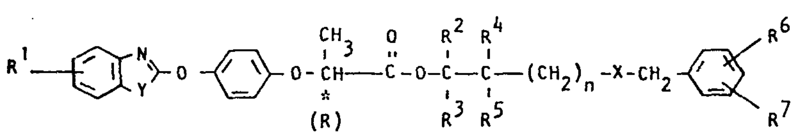

(I)

in welcher

$R^1$ für Wasserstoff oder Halogen,

Y für Sauerstoff oder Schwefel,

X für Sauerstoff, Schwefel, Sulfinyl oder Sulfonyl,

$R^2$,$R^3$,$R^4$ und $R^5$ für Wasserstoff oder geradkettiges oder verzweigtes Alkyl,

$R^6$ und $R^7$ für Wasserstoff, geradkettiges oder verzweigtes Alkyl, Halogen oder für einfach oder mehrfach, gleich oder verschieden durch Halogen substituiertes geradkettiges oder verzweigtes Alkyl und

n für 0, 1 oder 2

steht,

Verfahren zu deren Herstellung und ihre Verwendung als Herbizide.

EP 0 192 031 A1

Croydon Printing Company Ltd.

0192031

BAYER AKTIENGESELLSCHAFT 5090 Leverkusen, Bayerwerk

Konzernverwaltung RP KM/by-c
Patentabteilung Ia

# Optisch aktive Phenoxypropionsäure-Derivate

Die vorliegende Erfindung betrifft neue R-Enantiomere von Phenoxypropionsäure-Derivaten, mehrere Verfahren zu deren Herstellung und ihre Verwendung als Herbizide.

Es ist bereits bekannt, daß zahlreiche Phenoxypropionsäure-Derivate herbizide Eigenschaften besitzen (vgl. DE-OS 26 40 730). So können z.B. 2-(4-/(6-Chlor-2-benzthiazolyl)-oxy/-phenoxy)-propionsäure-ethylester und 2-(4-/(6-Chlor-2-benzoxazolyl)-oxy/-phenoxy)-propionsäure-ethylester zur Bekämpfung von Unkraut eingesetzt werden. Die Wirkung dieser Stoffe ist jedoch nicht immer völlig ausreichend.

Es wurden nun neue R-Enantiomere von Phenoxypropionsäure-Derivaten der Formel (I)

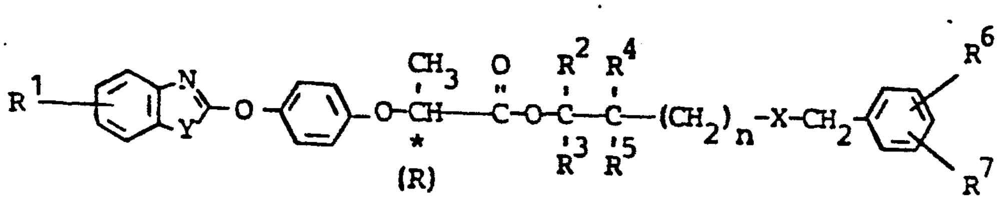

(I)

in welcher

$R^1$ für Wasserstoff oder Halogen,

Y für Sauerstoff oder Schwefel,

X für Sauerstoff, Schwefel, Sulfinyl oder Sulfonyl,

$R^2$,$R^3$,$R^4$ und $R^5$ jeweils unabhängig voneinander für Wasserstoff oder geradkettiges oder verzweigtes Alkyl,

$R^6$ und $R^7$ jeweils unabhängig voneinander für Wasserstoff, geradkettiges oder verzweigtes Alkyl, Halogen oder für einfach oder mehrfach, gleich oder verschieden durch Halogen substituiertes Alkyl und

n für 0, 1 oder 2

stehen,

gefunden.

In dieser Formel (I) ist das asymmetrisch substituierte Kohlenstoffatom durch ein (*) gekennzeichnet.

Weiterhin wurde gefunden, daß man die Phenoxypropionsäure-Derivate der Formel (I) erhält, wenn man

a) Verbindungen der Formel (II)

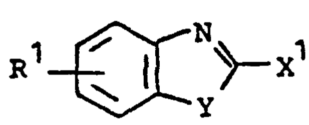

(II)

in welcher

$X^1$ für Halogen, $(C_1-C_4)$-Alkylsulfonyl, $(C_1-C_4)$-Alkylsulfinyl, $(C_1-C_4)$-Alkylthio, sowie für den Mesyl- oder Tosylatrest steht und

$R^1$ und Y die oben angegebene Bedeutung haben,

mit optisch aktiven Hydrochinon-Derivaten der Formel (III)

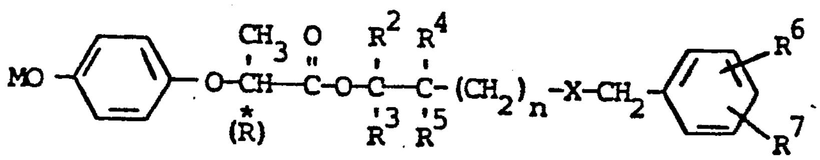

(III)

in welcher

M für Wasserstoff, ein Alkalimetallatom oder für das Erdalkalimetallatom Calcium steht und

$X, R^2, R^3, R^4, R^5, R^6, R^7$ und n die oben angegebene Bedeutung haben,

in Gegenwart eines Säurebindemittels sowie gegebenenfalls in Gegenwart eines Verdünnungsmittels umsetzt,

oder

b) Verbindungen der Formel (IV)

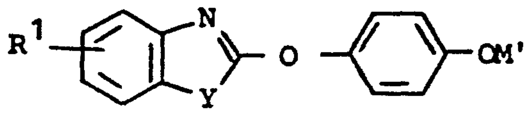

(IV)

in welcher

M' für Wasserstoff oder ein Alkalimetallatom steht und

$R^1$ und Y die oben angegebene Bedeutung haben,

mit S-Enantiomeren von Propionsäure-Derivaten der Formel (V)

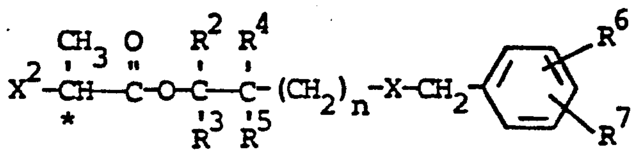

(V)

in welcher

$X^2$ für Halogen, $(C_1-C_4)$-Alkylsulfonyl, den Mesyl- oder Tosylatrest steht und

X, $R^2$, $R^3$, $R^4$, $R^5$, $R^6$, $R^7$ und n die oben angegebene Bedeutung haben,

in Gegenwart eines Säurebindemittels sowie gegebenenfalls in Gegenwart eines Verdünnungsmittels umsetzt.

Schließlich wurde gefunden, daß sich die erfindungsgemäßen R-Enantiomeren von Phenoxypropionsäure-Derivaten der Formel (I) durch starke herbizide Eigenschaften auszeichnen.

Überraschenderweise zeigen die neuen R-Enantiomeren von Phenoxypropionsäure-Derivaten der Formel (I) eine erheblich höhere herbizide Wirkung als der 2-(4-/(6-Chlor-2-benzoxazolyl)-oxy7-phenoxy-propionsäure-ethylester und der 2-(4-/6-Chlor-2-benzthiazolyl)-oxy7-phenoxy)-propionsäure-ethylester, welche konstitutionell ähnliche vorbekannte Verbindungen gleicher Wirkungsart sind.

Die erfindungsgemäßen R-Enantiomeren von Phenoxypropionsäure-Derivaten sind durch die Formel (I) allgemein definiert. In dieser Formel stehen

$R^1$ vorzugsweise für Wasserstoff oder Chlor oder Brom,

Y für Sauerstoff oder Schwefel,

X für Sauerstoff, Schwefel, Sulfinyl oder Sulfonyl,

$R^2$,$R^3$,$R^4$ und $R^5$ vorzugsweise für Wasserstoff oder geradkettiges oder verzweigtes Alkyl mit 1 bis 4 Kohlenstoffatomen,

$R^6$ und $R^7$ vorzugsweise für Wasserstoff, geradkettiges oder verzweigtes Alkyl mit 1 bis 4 Kohlenstoffatomen, Fluor, Chlor, Brom oder für einfach oder mehrfach, gleich oder verschieden durch Fluor, Chlor oder Brom substituiertes geradkettiges oder verzweigtes Alkyl mit 1 bis 4 Kohlenstoffatomen und

n für 0, 1 oder 2.

Besonders bevorzugt sind diejenigen Verbindungen der Formel (I), in denen

$R^1$ für Wasserstoff oder Chlor,

Y für Sauerstoff oder Schwefel,

X für Sauerstoff, Schwefel, Sulfinyl oder Sulfonyl,

$R^2$,$R^3$,$R^4$ und $R^5$ für Wasserstoff, Methyl oder Ethyl,

$R^6$ und $R^7$ für Wasserstoff, Methyl, Ethyl, Fluor, Chlor, Trifluormethyl, Trichlormethyl, Dichlorfluormethyl, Difluorchlormethyl, Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl und

n für 0, 1 oder 2 steht.

0192031

Verwendet man 2,6-Dichlor-benzoxazol und (Benzyloxy)-ethyl-2-(4-hydroxyphenoxy)-propionat als Ausgangsstoffe, so kann der Reaktionsablauf des erfindungsgemäßen Verfahrens (a) durch das folgende Reaktionsschema wiedergegeben werden:

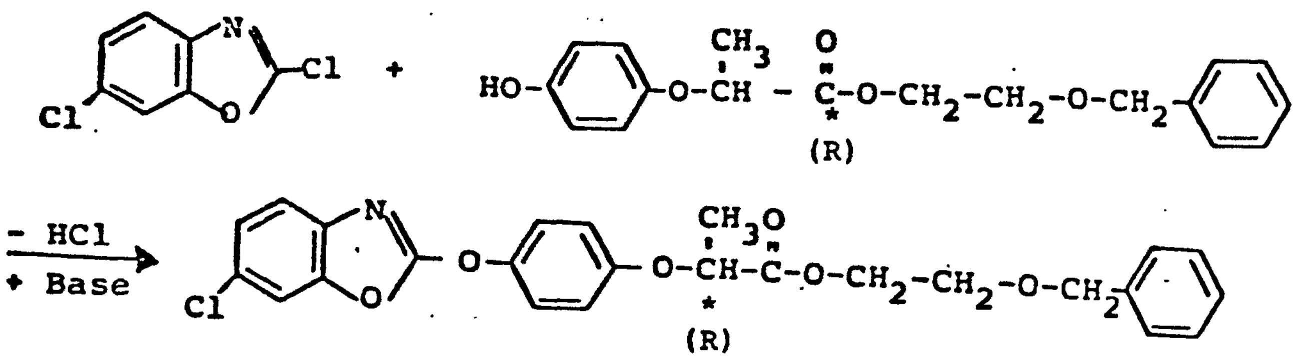


Verwendet man 4-[(5-Chlor-benzoxazolyl)-oxy]-phenol und 2-Brom-propionsäure-(2-benzyloxy)-ethylester als Ausgangsstoffe, so kann der Reaktionsablauf des erfindungsgemäßen Verfahrens (b) durch das folgende Reaktionsschema wiedergegeben werden:

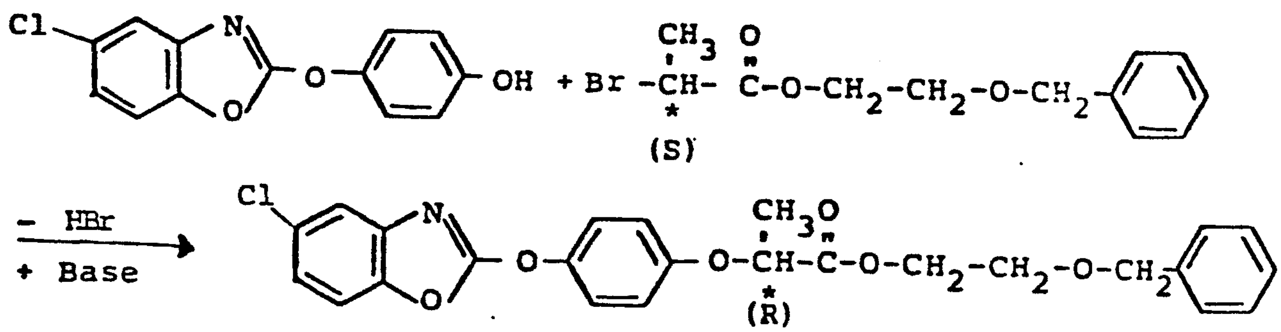

Die bei dem erfindungsgemäßen Verfahren (a) als Ausgangsstoffe benötigten Verbindungen sind durch die Formel (II) definiert. In dieser Formel steht $X^1$ vorzugsweise für Chlor, Brom, Methylsulfonyl, Ethylsulfonyl oder Tosylat, $R^1$ und Y haben vorzugsweise diejenigen Bedeutungen, die bereits im Zusammenhang mit der Beschreibung der erfindungsgemäßen Stoffe der Formel (I) für diese Reste genannt wurden. Die Verbindungen der Formel (II) sind bekannt (vgl. DE-OS 2 640 730, EP-OS 0 044 497, EP-OS 0 075 840).

Die bei dem erfindungsgemäßen Verfahren (a) weiterhin als Ausgangsstoffe benötigten optisch aktiven Hydrochinon-Derivate sind durch die Formel (III) eindeutig definiert. In dieser Formel steht M vorzugsweise für Wasserstoff, die Alkalimetallatome Natrium oder Kalium oder das Erdalkalimetallatom Kalcium; X, $R^2$, $R^3$, $R^4$, $R^5$, $R^6$, $R^7$ und n besitzen in dieser Formel diejenigen Bedeutungen, die bereits im Zusammenhang mit der Beschreibung der erfindungsgemäßen Stoffe der Formel (I) vorzugsweise für diese Reste bzw. für diesen Index genannt wurden. Diese optisch aktiven Hydrochinon-Derivate der Formel (III) lassen sich nach prinzipiell bekannten Verfahren in einfacher Weise herstellen (vgl. DE-OS 27 58 002, EP 00 68 260).

Die bei dem erfindungsgemäßen Verfahren (b) als Ausgangsstoffe benötigten Verbindungen sind durch die Formel (IV) definiert. Die Verbindungen der Formel (IV) sind bekannt

oder lassen sich nach bekannten Verfahren herstellen (vgl. DE-OS 33 11 285, J. Am. Chem. Soc. 954-973 (1965), EP 00 03 562).

Die bei dem erfindungsgemäßen Verfahren (b) weiterhin als Ausgangsstoffe benötigten optisch aktiven Propionsäure-Derivate sind durch die Formel (V) allgemein definiert. In dieser Formel steht $X^2$ vorzugsweise für Chlor, Brom, Methylsulfonyl, Ethylsulfonyl, Mesyl oder Tosylat; $R^2$, $R^3$, $R^4$, $R^5$, $R^6$, $R^7$, X und n haben in dieser Formel vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden. Die optisch aktiven Propionsäure-Derivate der Formel (V) sind bekannt (vgl. DE-OS 32 47 930) oder lassen sich nach prinzipiell bekannten Verfahren herstellen.

Die erfindungsgemäßen Verfahren (a) und (b) zur Herstellung der neuen R-Enantiomeren von Phenoxypropionsäure-Derivaten der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether,

wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon sowie Dimethylsulfoxid, Tetramethylensulfon und Hexamethylphosphorsäuretriamid.

Als Säurebindemittel können bei den erfindungsgemäßen Verfahren (a) und (b) alle üblicherweise für derartige Umsetzungen verwendbaren Säurebindemittel eingesetzt werden. Vorzugsweise infrage kommen Alkalihydroxide, wie z.B. Natrium- und Kaliumhydroxid, Erdalkalihydroxyde, z.B. Calciumhydroxyd, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin 1,5-Diaza-bicyclo-[4.3.0]-nonen-5 (DBN) und 1,8-Diazabicyclo-[5.4.0]-undec-7-en (DBU).

Die Reaktionstemperaturen können bei den erfindungsgemäßen Verfahren (a) und (b) innerhalb eines größeren Bereiches variiert werden. Im allgemeinen arbeitet man jeweils bei Temperaturen zwischen -20°C und +160°C, vorzugsweise zwischen 0°C und 140°C.

Die erfindungsgemäßen Verfahren (a) und (b) werden im allgemeinen unter Normaldruck durchgeführt. Es ist

jedoch auch möglich, unter erhöhtem oder vermindertem Druck zu arbeiten.

Zur Durchführung der erfindungsgemäßen Verfahren (a) und (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a) und (b) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium,

Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne

Baumbewuchs. Ebenso kann die Verbindung zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich sehr gut zur selektiven Unkrautbekämpfung in Getreide und Reis. Sie besitzen vor allem eine sehr gute Wirkung gegen grasartige Unkräuter.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und

chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie

Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z.B. N-(2-Benzthiazolyl)-N,N'-dimethyl-harnstoff, 3-(3-Chlor-4-methylphenyl)-1,1-dimethylharnstoff, 3-(4-Isopropylphenyl)-1,1-dimethylharnstoff, 4-Amino-6-(1,1-dimethylethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on; 2-Chlor-4-ethylamino-6-isopropyl-amino-1,3,5-triazin, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-3-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

0192031

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die vorliegende Erfindung wird durch die folgenden Beispiele näher erläutert.

Herstellungsbeispiel

Beispiel 1 nach Verfahren (b)

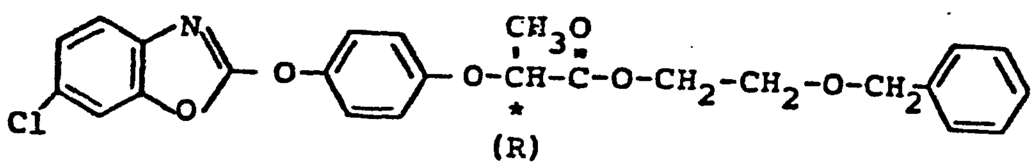

Eine Lösung von 5,2 g (0,02 Mol) 4-(6-Chlor-2-benzoxazolyloxy)-phenol in 50 ml Acetonitril wird mit 2,7 g (0,02 Mol) Kaliumcarbonat versetzt. Bei 20°C tropft man eine Lösung von 8 g (0,02 Mol) des S-Enantiomeren des Tosyloxypropionsäure-(2-benzyloxy)-ethylester in 10 ml Acetonitril zu. Anschließend wird 3 Stunden unter Rückfluß erhitzt. Zur Aufarbeitung wird die Reaktionslösung mit Wasser versetzt und das anfallende ölige Produkt mit Chloroform extrahiert. Die organische Phase wird nacheinander mit 2 n wäßriger Natronlauge und mit schwach salzsaurem Wasser gewaschen. Nach dem Trocknen wird das Verdünnungsmittel unter vermindertem Druck eingeengt. Der Rückstand wird in Diisopropylether aufgenommen. Danach wird filtriert und das Verdünnungsmittel unter vermindertem Druck abgezogen.

Man erhält auf diese Weise 7 g (75 % der Theorie) an dem R-Enantiomeren des 2-/4-(6-Chlor-2-benzoxazolyloxy-phenoxy/-propionsäure-(2-benzyloxy)-ethylester als öliges Produkt.

Brechungsindex: $n_D^{20} = 1,5604$

Drehwert: $[\alpha]_D^{25} = + 14,2$

Beispiel 2 nach Verfahren (b)

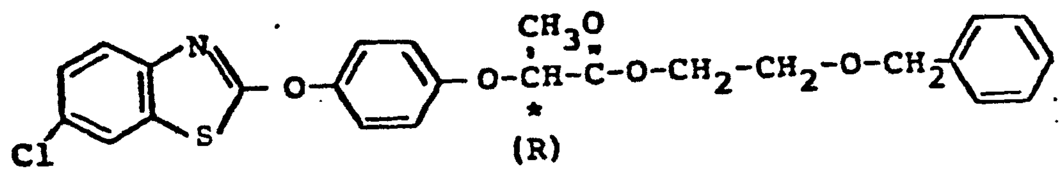

Eine Lösung von 5,6 g (0,02 Mol) 4-(6-Chlor-2-benzthiazolyloxy)-phenol in 50 ml Acetonitril wird mit 2,7 g (0,02 Mol) Kaliumcarbonat versetzt. Bei 20°C tropft man eine Lösung von 8 g (0,02 Mol) des S-Enantiomeren des Tosyloxypropionsäure-(2-benzyloxy)-ethylester in 10 ml Acetonitril zu. Anschließend wird 3 Stunden unter Rückfluß erhitzt. Zur Aufarbeitung wird die Reaktionslösung mit Wasser versetzt und das anfallende ölige Produkt mit Chloroform extrahiert. Die organische Phase wird nacheinander mit 2 n wäßriger Natronlauge und mit schwach salzsaurem Wasser gewaschen. Nach dem Trocknen wird das Verdünnungsmittel unter vermindertem Druck eingeengt. Der Rückstand wird in Diisopropylether aufgenommen. Danach wird filtriert und das Verdünnungsmittel unter vermindertem Druck abgezogen.

Man erhält auf diese Weise 6,8 g (70 % der Theorie) an dem R-Enantiomeren des 2-/4-(6-Chlor-2-benzthiazolyloxy-phenoxy/-propionsäure-(2-benzyloxy)-ethylester als öliges Produkt.

Brechungsindex: $n_D^{20} = 1,5775$

Drehwert: $[\alpha]_D^{25} = + 12,4$

Nach den in den Herstellungsbeispielen beschriebenen Methoden lassen sich auch die in der folgenden Tabelle 1 aufgeführten Stoffe herstellen.

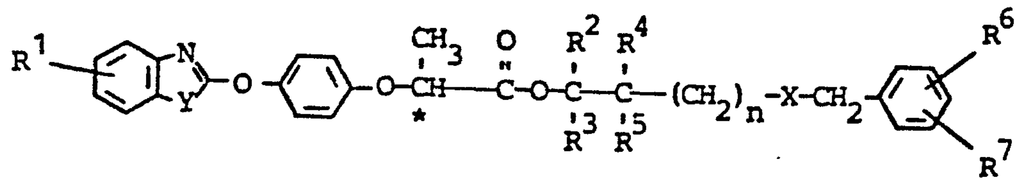

Tabelle 1

| Bsp. Nr. | $R^1$ | Y | $R^2$ | $R^3$ | $R^4$ | $R^5$ | n | X | $R^6$ | $R^7$ | Drehwert $[\alpha]_D^{33}$ |
|---|---|---|---|---|---|---|---|---|---|---|---|
| 3 | 6-Cl | O | H | H | $CH_3$ | $CH_3$ | 1 | O | H | H | |
| 4 | 6-Cl | O | H | H | H | H | 1 | S | H | H | |
| 5 | 6-Cl | O | H | H | H | H | 1 | SO | H | H | |
| 6 | 6-Cl | O | H | H | H | H | 1 | $SO_2$ | H | H | |
| 7 | 6-Cl | O | H | H | H | H | 0 | 0 | H | 4-Cl | |
| 8 | 6-Cl | O | H | H | H | H | 1 | 0 | H | H | |
| 9 | 6-Cl | O | $CH_3$ | H | $CH_3$ | H | 0 | 0 | H | H | |
| 10 | 6-Cl | O | H | H | H | H | 0 | 0 | H | 4-$CF_3$ | |
| 11 | 5-Cl | O | H | H | $CH_3$ | $CH_3$ | 0 | 0 | H | H | |
| 12 | 5-Cl | O | H | H | H | H | 0 | 0 | H | H | |
| 13 | 5-Cl | O | H | H | H | H | 0 | S | H | H | |
| 14 | 6-Cl | O | H | H | H | H | 0 | 0 | H | 2-F | |
| 15 | 6-Cl | O | H | H | H | H | 0 | 0 | H | 2-Cl | +13,22 |
| 16 | 6-Cl | O | H | H | H | H | 0 | 0 | H | 4-$CH_3$ | +14,37 |
| 17 | 6-Cl | O | H | H | $CH_3$ | $CH_3$ | 1 | 0 | H | H | |

Herstellung von Ausgangssubstanzen:

Beispiel (IV-1)

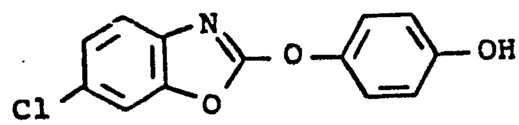

Ein Gemisch aus 99,4 g (0,9 Mol) Hydrochinon, 900 ml Dimethylsulfoxid und 3,4 g (0,45 Mol) Calciumhydroxid wird unter Stickstoff auf 80°C erhitzt.Danach werden unter Rühren bei dieser Temperatur 85 g (0,045 Mol) 2,6-Dichlorbenzoxazol, die im beheizten Tropftrichter flüssig gehalten werden, langsam zugetropft. Anschließend wird aufgearbeitet, indem man das Reaktionsgemisch durch Abziehen des Lösungsmittels einengt, den verbleibenden Rückstand mit Wasser und wäßriger Salzsäure versetzt und das ausfallende Festprodukt absaugt und trocknet. Man erhält auf diese Weise 100 g (85 % der Theorie) an 4-(6-Chlor-2-benzoxazolyl)-oxy-phenol.

Schmelzpunkt: 176°C (umkristallisiert aus Butanol)

Beispiel (IV-2)

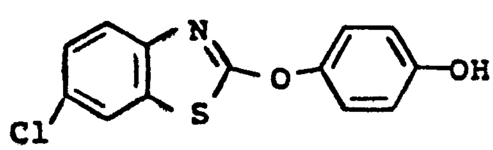

(IV-2)

Ein Gemisch aus 11 g (0,1 Mol) Hydrochinon, 50 ml Dimethylsulfoxid und 3,7 g (0,05 Mol) Calciumhydroxid wird unter Stickstoff auf 80°C erhitzt. Danach wird unter Rühren bei dieser Temperatur eine Lösung von 10,2 g (0,05 Mol) 2,6-Dichlorbenzthiazol in 10 ml Dimethylsulfoxid langsam zugetropft. Es wird 8 Stunden bei 80°C nachgerührt. Anschließend wird aufgearbeitet, indem man das Reaktionsgemisch durch Abziehen des Lösungsmittels einengt, den verbleibenden Rückstand mit Wasser und wäßriger Salzsäure versetzt und das ausgefallene Festprodukt absaugt und trocknet. Man erhält auf diese Weise 11,1 g (80 % der Theorie) an 4-(6-Chlor-2-benzthiazolyl)-oxyphenol.

Schmelzpunkt: 174°C (nach Umkristallisation aus Xylol).

Beispiel (IV-3)

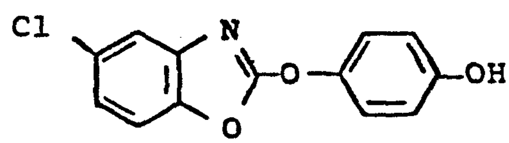

(IV-3)

Nach der in den Beispielen (IV-1) und (IV-2) beschriebenen Methode wird auch die Verbindung der Formel (IV-3) hergestellt.

Ausbeute: 90 % der Theorie
Schmelzpunkt: 176-177°C

Beispiel (V-1)

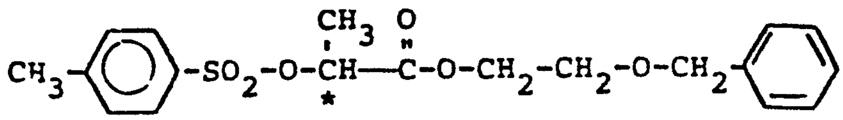

(V-1)

5,25 g (0,02 Mol) des linksdrehenden Enantiomeren des Milchsäurechlorid-tosylats wurden bei 20°C unter Rühren in ein Gemisch aus 3,32 g (0,02 Mol) Glykol-mono-benzylether, 2 g (0,02 Mol) Triethylamin und 50 ml Toluol gegeben. Man rührte weitere 14 Stunden bei 70°C und arbeitete dann auf, indem man das Reaktionsgemisch mit 100 ml Wasser versetzte, dann mehrfach mit Toluol extrahierte, die vereinigten organischen Phasen trocknete und durch Abziehen des Lösungsmittels unter vermindertem Druck einengte. Man erhielt auf diese Weise 6,3 g (80,5 % der Theorie) an linksdrehendem Enantiomeren des Tosyloxy-propion-säure-(2-benzyloxy)-ethylesters.

Drehwert: $[\alpha]_D^{24}$ = -10,2°
(1-molare Lösung in Chloroform;
Küvettenlänge = 10 cm).

S/R 96:4

Nach der im Beispiel (V1) beschriebenen Methode lassen sich auch die in der folgenden Tabelle 2 aufgeführten Stoffe herstellen:

Tabelle 2:

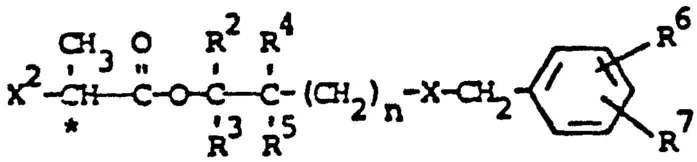

(V)

| Bsp. Nr. | $X^2$ | $R^2$ | $R^3$ | $R^4$ | $R^5$ | n | X | $R^6$ | $R^7$ | Drehwert $[\alpha]_D^{24}$ |
|---|---|---|---|---|---|---|---|---|---|---|
| V-2 | Tosylat | H | H | H | H | 0 | 0 | 2-F | H | - 9,7 |
| V-3 | Tosylat | H | H | H | H | 1 | 0 | H | H | -10,3 |
| V-4 | Tosylat | H | $CH_3$ | H | H | 0 | 0 | H | H | - 4,1 |
| V-5 | Tosylat | H | H | H | H | 0 | 0 | 4-Cl | H | - 7,6 |
| V-6 | Tosylat | H | H | H | H | 0 | S | H | H | -10,9 |
| V-7 | Tosylat | H | H | H | H | 0 | 0 | 2-Cl | H | -45,36 |
| V-8 | Tosylat | H | H | $CH_3$ | $CH_3$ | 1 | 0 | H | H | |
| V-9 | Tosylat | H | H | H | H | 0 | 0 | 4-$CH_3$ | H | -24,92 |

In den nachstehend beschriebenen biologischen Tests wurden die folgenden Verbindungen als Vergleichssubstanzen eingesetzt:

(A)

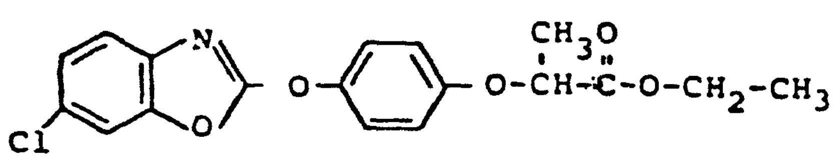

2-(4-/¯(6-Chlor-2-benzoxazolyl)-oxy7-phenoxy)-propionsäure-ethylester
(bekannt aus DE-OS 2 640 730).

(B) 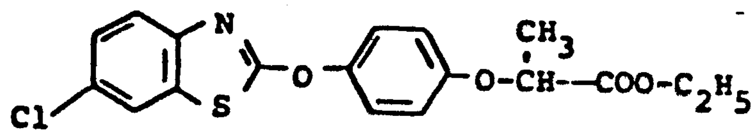

2-{4-[(6-Chlor-2-benzthiazolyl)-oxy]-phenoxy}-propionsäure-ethylester
(bekannt aus DE-OS 2 640 730).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt der erfindungsgemäße Wirkstoff des Beispiels 1 bei der Bekämpfung von Digitaria, Setaria und Panicum in Soja, Zuckerrüben und Weizen eine deutlich selektivere Wirkung als die Vergleichssubstanz (A).

0192031

Patentansprüche

1. R-Enantiomere von Phenoxypropionsäure-Derivaten der Formel (I)

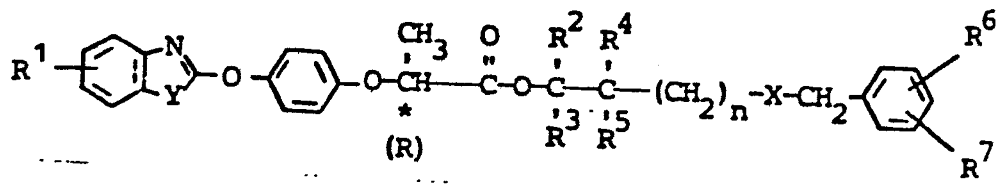

(I)

in welcher

$R^1$ für Wasserstoff oder Halogen,

Y für Sauerstoff oder Schwefel,

X für Sauerstoff, Schwefel, Sulfinyl oder Sulfonyl,

$R^2$, $R^3$, $R^4$ und $R^5$ für Wasserstoff oder geradkettiges oder verzweigtes Alkyl,

$R^6$ und $R^7$ für Wasserstoff, geradkettiges oder verzweigtes Alkyl, Halogen oder für einfach oder mehrfach, gleich oder verschieden durch Halogen substituiertes geradkettiges oder verzweigtes Alkyl und

n    für 0, 1 oder 2

steht.

2. R-Enantiomere von Phenoxypropionsäure-Derivaten der Formel (I), gemäß Anspruch 1 in denen

$R^1$ für Wasserstoff oder Chlor oder Brom,

Y für Sauerstoff oder Schwefel,

X für Sauerstoff, Schwefel, Sulfinyl oder Sulfonyl,

$R^2$,$R^3$,$R^4$ und $R^5$ für Wasserstoff oder geradkettiges oder verzweigtes Alkyl mit 1 bis 4 Kohlenstoffatomen,

$R^6$ und $R^7$ für Wasserstoff, geradkettiges oder verzweigtes Alkyl mit 1 bis 4 Kohlenstoffatomen, Fluor, Chlor, Brom oder für einfach oder mehrfach, gleich oder verschieden durch Fluor, Chlor oder Brom substituiertes geradkettiges oder verzweigtes Alkyl mit 1 bis 4 Kohlenstoffatomen und

n    für 0, 1 oder 2

steht.

3. R-Enantiomere von Phenoxypropionsäure-Derivaten der Formel (I), gemäß Anspruch 1 in denen

$R^1$ für Wasserstoff oder Chlor,

Y für Sauerstoff oder Schwefel,

X für Sauerstoff, Schwefel, Sulfinyl oder Sulfonyl,

$R^2$,$R^3$,$R^4$ und $R^5$ für Wasserstoff, Methyl oder Ethyl,

$R^6$ und $R^7$ für Wasserstoff, Methyl, Ethyl, Fluor, Chlor, Trifluormethyl, Trichlormethyl, Dichlorfluormethyl, Difluorchlormethyl, Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl und

n für 0, 1 oder 2

stehen.

4. Verfahren zur Herstellung von R-Enantiomeren von Phenoxypropionsäure-Derivaten der Formel (I)

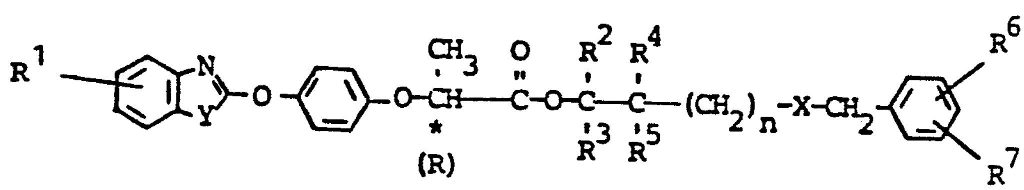

(I)

in welcher

$R^1$ für Wasserstoff oder Halogen,

Y für Sauerstoff oder Schwefel,

X für Sauerstoff, Schwefel, Sulfinyl oder Sulfonyl,

$R^2$,$R^3$,$R^4$ und $R^5$ für Wasserstoff oder geradkettiges oder verzweigtes Alkyl,

$R^6$ und $R^7$ für Wasserstoff, geradkettiges oder verzweigtes Alkyl, Halogen oder für einfach oder mehrfach, gleich oder verschieden durch Halogen substituiertes geradkettiges oder verzweigtes Alkyl und

n für 0,1 oder 2

steht,

dadurch gekennzeichnet, daß man

a) Verbindungen der Formel (II)

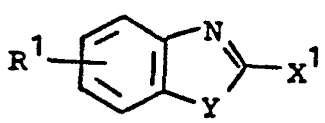

(II)

in welcher

$X^1$ für Halogen, $(C_1-C_4)$-Alkylsulfonyl, $(C_1-C_4)$-Alkylsulfinyl, $(C_1-C_4)$-Alkylthio, sowie für den Mesyl- oder Tosylatrest steht und

$R^1$ und Y die oben angegebene Bedeutung haben,

mit optisch aktiven Hydrochinon-Derivaten der Formel (III)

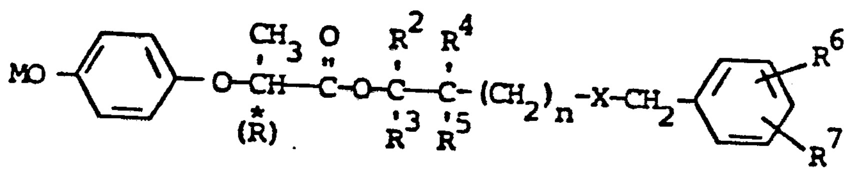

(III)

in welcher

M für Wasserstoff, ein Alkalimetallatom oder für das Erdalkalimetallatom Calcium steht und

$X, R^2, R^3, R^4, R^5, R^6, R^7$ und n die oben angegebene Bedeutung haben,

in Gegenwart eines Säurebindemittels sowie gegebenenfalls in Gegenwart eines Verdünnungsmittels umsetzt,

oder

b) Verbindungen der Formel (IV)

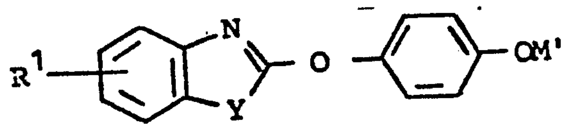

(IV)

in welcher

M' für Wasserstoff oder ein Alkalimetallatom steht und

$R^1$ und Y die oben angegebene Bedeutung haben,

mit S-Enantiomeren von Propionsäure-Derivaten der Formel (V)

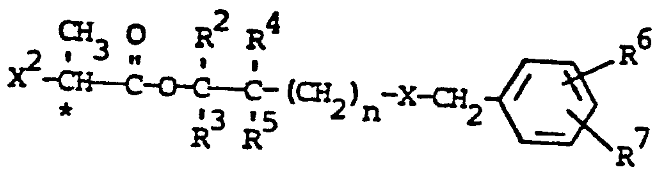

(V)

in welcher

$X^2$ für Halogen, $(C_1-C_4)$-Alkylsulfonyl, sowie für den Mesyl- oder Tosylatrest steht und

$X, R^2, R^3, R^4, R^5, R^6, R^7$ und n die oben angegebene Bedeutung haben,

in Gegenwart eines Säurebindemittels sowie gegebenenfalls in Gegenwart eines Verdünnungsmittels umsetzt.

5. Herbizide Mittel, gekennzeichnet durch einen Gehalt an mindestens einem R-Enantiomeren von Phenoxypropionsäure-Derivaten der Formel (I) gemäß den Ansprüchen 1 bis 4.

6. Verfahren zur Bekämpfung von Unkräutern, dadurch gekennzeichnet, daß man R-Enantiomere von Phenoxypropionsäure-Derivaten der Formel (I) gemäß den Ansprüchen 1 bis 4 auf die Unkräuter und/oder ihren Lebensraum ausbringt.

7. Verwendung von R-Enantiomeren von Phenoxypropionsäure-Derivaten der Formel (I) gemäß den Ansprüchen 1 bis 4 zur Bekämpfung von Unkräutern.

8. Verfahren zur Herstellung von herbiziden Mitteln, dadurch gekennzeichnet, daß man R-Enantiomere von Phenoxypropionsäure-Derivaten der Formel (I) gemäß den Ansprüchen 1 bis 4 mit Streckmitteln und/oder oberflächenaktiven Stoffen vermischt.

0192031

Europäisches Patentamt

# EUROPÄISCHER RECHERCHENBERICHT

Nummer der Anmeldung

EP 86 10 0263

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | EP-A-0 002 800 (HOECHST AKTIENGESELLSCHAFT) * Seite 2, Zeile 12 - Seite 6, Zeile 32; Seite 10, Zeile 21 - Seite 11, Zeile 7 * & DE - A - 2 758 002 (Kat. D) | 1-8 | C 07 D 263/58<br>C 07 D 277/68<br>A 01 N 43/76<br>A 01 N 43/78 //<br>C 07 C 143/68 |
| | --- | | |
| Y | EP-A-0 112 531 (BAYER AG) * Seite 2, Zeile 1 - Seite 3, Zeile 13; Seite 4, Zeile 10 - Seite 5, Zeile 12 * & DE - A - 3 247 930 (Kat. D) | 1-8 | |
| | --- | | |
| A | DE-A-3 018 003 (HOECHST AG) * Seite 4, Zeile 1 - Seite 7, Zeile 17; Seite 9, Zeilen 2-31 * | 1,4-8 | |
| | --- | | RECHERCHIERTE SACHGEBIETE (Int. Cl.4) |
| A,D | EP-A-0 044 497 (HOECHST AKTIENGESELLSCHAFT) * Seite 1, Zeile 1 - Seite 3, Zeile 26, Seite 4, Zeilen 28-32 * | 1,4,5 | C 07 D 263/00<br>C 07 D 277/00<br>A 01 N 43/00<br>A 01 N 39/00 |
| | --- | | |
| P,X | EP-A-0 141 319 (NIHON TOKUSHU NOYAKU SEIZO K.K.) * Ansprüche, Seite 11, Zeile 4 - Seite 12, Zeile 4 * | 1-8 | |
| | ----- | | |

Der vorliegende Recherchenbericht wurde für alle Patentansprüche erstellt

| Recherchenort | Abschlußdatum der Recherche | Prüfer |
|---|---|---|
| BERLIN | 05-05-1986 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82